# EUROPEAN PATENT SPECIFICATION

(11) **EP 3 985 382 B1**
(45) Date of publication and mention of the grant of the patent: **25.11.2026**
(21) Application number: 20201896.6
(22) Date of filing: 14.10.2020
(51) Int. Cl.: G01N 21/84, H04N 17/00, H04N 23/61, H04N 23/71, H04N 23/73, H04N 23/75, H04N 23/76, A61B 10/00

(54) **A METHOD OF CONTROLLING AUTO-EXPOSURE SETTINGS OF A MOBILE DEVICE HAVING A CAMERA**
VERFAHREN ZUR STEUERUNG VON AUTOMATISCHEN BELICHTUNGSEINSTELLUNGEN EINER MOBILEN VORRICHTUNG MIT EINER KAMERA
PROCÉDÉ DE COMMANDE DE RÉGLAGES D'EXPOSITION AUTOMATIQUE D'UN DISPOSITIF MOBILE DOTÉ D'UNE CAMÉRA

(43) Date of publication of application: 20.04.2022
(73) Proprietor: Roche Diabetes Care GmbH, 68305 Mannheim (DE); F. Hoffmann-La Roche AG, 4070 Basel (CH)
(72) Inventor: KOSCHORRECK, Beate, 68305 Mannheim (DE); LIMBURG, Bernhard, 68305 Mannheim (DE); SCHMIDT, Guenter, 68305 Mannheim (DE); SCHWOBEL, Wolfgang, 68305 Mannheim (DE); BERG, Max Herrmann, 68305 Mannheim (DE); HAILER, Fredrik, 68305 Mannheim (DE); MELCHINGER, Christian, 68305 Mannheim (DE)
(74) Representative: Wandrey, Anne Julia

(56) References cited:
- WO-A1-2015/038717
- US-A1- 2018 146 175
- US-A1- 2020 126 226

## Description

### Technical Field

The present invention relates to a method of determining the concentration of an analyte in a sample by using a mobile device. The methods, devices, computer program and storage mediun disclosed herein specifically may be used in medical diagnostics, in order to for example qualitatively and/or quantitatively detect one or more analytes in one or more body fluids, such as for detecting glucose in blood and/or interstitial fluid. Other fields of application of the present invention, however, are also feasible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test strips.

Generally, devices and methods known to the skilled person make use of test strips comprising one or more test chemistries, which, in presence of the analyte to be detected, are capable of performing one or more detectable detection reactions, such as optically detectable detection reactions. With regard to these test chemistries, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

Typically, one or more optically detectable changes in the test chemistry are monitored, in order to derive the concentration of the at least one analyte to be detected from these changes. For detecting the at least one change of optical properties of the test field of a test strip, various types of detectors (with various types of light sources for illuminating the test fields) are known (also referred to as dedicated meters or analytical measurement devices).

Further, besides using customized detectors which are specifically developed for the purpose of optically detecting changes in the test chemistry comprised by corresponding test strips, recent developments aim at using widely available devices such as smartphones. However, when consumer-electronics devices having a camera, such as smartphones, are employed instead of dedicated analytical measurement devices in order to determine analyte concentrations various influences need to be taken into account. As an example, lighting conditions, positioning, or other more or less uncontrollable conditions are to be considered.

US20170042451A1 describes that pixel values in an image taking with a computing device which can be a smartphone are heavily dependent on the lighting that existed when the image was taken, and on the exposure of the image taken by the camera. It is therefore suggested to take an image with a reference card also visible in the image. A location on the card having a pure gray color of a known reflectivity is then identified in the image that was taken and pixels on this card location are analyzed in order to standardize both the exposure and the white balance of the image. The standardization is then applied to an average value of selected pixels of the image, in order to create a corrected pixel value.

US 10469807B2 describes a system for determining a color match using a template card comprising at least one reference scale. An image of the template card and a color sample is received, and color correction is applied to the sample RGB value based on a difference between the at least one reference scale RGB value and a corresponding calibration RGB value of the at least one reference scale to produce a color corrected sample RGB value. The color corrected sample RGB value is then compared with the RGB values of the color definitions stored within a database to determine a color match. It is described that an unknown camera gain and lighting effects may not affect the true red, green, and blue color values because the camera gain and lighting effects affect the template card in the same manner as they affect a sample of the unknown color. It is thus described that the template card serves as a reference card to eliminate the many variations that can impact the perception or imaging of an unknown color sample. It if further described that where the background color of the card is uniform and known, one can utilize this fact to compute and subtract out of any smooth nonuniformity, resulting in a residual nonuniformity several times smaller than the actual. This may be done by sampling a number of points in the image of the template background, which are known to be the same color, and determining the variance in the image from the known color based on the location of the point to find and remove smooth nonuniformity.

US9903857B2 describes a testing apparatus for performing an assay, the testing apparatus comprising: a receptacle containing a reagent, the reagent being reactive to an applied test sample by developing a color or pattern variation; a mobile phone or a laptop, comprising a processor and an image capture device, wherein the processor is configured to process data captured by the image capture device and output a test result for the applied test sample. It is further described that where the dynamic range of the resulting image is inadequate for the limit of detection, or where the constraints on lighting mean exposure settings force the dynamic range to be too poor it may be desirable to capture multiple images at different exposure settings and then combine these into a single higher dynamic range 'virtual' image, using appropriate algorithms to reorient the images between frames, and to discard unwanted or low value data. Contrasting colors, e.g. on the test strip housing, and distinct shapes of housing are described to simplify the image processing. Where there is no housing or the housing is a similar color to the test strip it is disclosed that it may be preferable to place the test strip against a contrasting background during image capture.

US9506855B2 discloses that a challenge for everyday use outside of a controlled setting for mobile phones for quantitative colorimetric analysis is compensating for ambient light conditions. Is it described that when measuring 12 regions on a reference chart, it was found that the measured intensities between different ambient light conditions had a linear relationship. It is concluded that the reference color chart can be used for compensating the intensity difference caused by ambient light changes. It is also described that the reference chart nearly eliminates effects of automatic camera functions, making images reproducible and quantifiable. Skewing the reference chart in the direction of warm colors by including yellow, orange, and red regions overcomes the tendency of AWB to decrease gain in the red channel of the CMOS imaging array, since silicon exhibits better responsiveness at these longer wavelengths. US 2020/126226 A1 discloses a method for analyzing visible chemical reactions. The method comprises receiving from an image sensor associated with a mobile communications device an image of a reagent pad in proximity to a colorized surface having at least one pair of colored reference elements designed to be used together to determine an extent of a reaction on the reagent pad, identifying in the image the reagent pad, identifying in the image a first colored reference element, identifying in the image a colored reference element, and using the first colored reference element and the second colored reference element to determine an extent of a chemical reaction on the reagent pad irrespective of local illumination conditions.

Despite the advantages involved in using a mobile computing device and a color reference card for the purpose of performing an analytical measurement, several technical challenges remain. Specifically, the use of mobile devices to determine analyte concentrations using optical test strips may require an accurate determination of the color change of the optical test strip and, thus, often remains challenging. Appropriate image brightness needs to be ensured. The observed brightness in the captured images may, e.g., be dependent on various influencing factors, such as settings determining the exposure when capturing the image, such as the shutter speed, the aperture size, and sensor gain, as well as post-processing steps applied to the image after it was captured. Specifically, the determination of the exposure settings by the mobile devices is often a device-specific process conducted in an automatic exposure (auto-exposure) mode and, thus, may be unknown to the manufacturer of the measurement application. Although an image taken with auto-exposure mode normally has a good overall or average brightness, there may be local under- or overexposures of image portions being much darker or much lighter than the image average. Such intensity values falling outside a minimum or maximum intensity which can be represented will appear as uniform area of the minimum or maximum brightness in the image so that image details are lost. If essential image elements (e.g., the test field) are thus within those under- or overexposed image areas, this image cannot be used for determining an analyte concentration.

### Problem to be solved

It is therefore desirable to provide methods and devices which address the above mentioned technical challenges using mobile devices such as consumer-electronics mobile devices, specifically multipurpose mobile devices which are not dedicated to analytical measurements such as smartphones or tablet computers. Specifically, methods and devices shall be proposed for controlling auto-exposure settings of a mobile device to ensure acquisition of images suitable for reliable and accurate determination of an analyte concentration. It is further desirable to provide a method and devices which allow for a user-friendly mobile-based determination of the analyte in the sample of the bodily fluid, with high accuracy and reliability of the analytical measurement, and, thus, may take into account image brightness and device-specific aspects.

### Summary

This problem is addressed by the invention as defined in claim 1.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The invention provides a method of determining the concentration of an analyte in a sample by using a mobile device having at least one camera,
the method comprising the following step:
i) controlling auto-exposure settings of the mobile device when capturing, by using the camera, at least one image of at least part of a color reference card and of at least part of a reagent test field of an optical test strip having a sample applied thereto, comprising
   a) providing a color reference card and an optical test strip having a test field having a sample applied thereto,
      ∘ wherein the color reference card comprises a plurality of different color reference fields having known reference color values and one or more gray background fields having defined gray values,
   b) setting an exposure metering area and determining auto-exposure settings based on the scene in the set exposure metering area, wherein the exposure metering area is set to comprise just a part in the center of the field of view of the camera,
      ∘ wherein the scene in the set exposure metering area comprises at least part of the reagent test field of the optical test strip having the sample applied thereto and the entire the color reference card, and wherein determining the auto-exposure settings is automatically initiated once the presence of at least one object within a predetermined part of the field of view of the camera is automatically detected, the object comprising the color reference card,
   c) capturing, by using the camera, an image comprising the scene of step b),
      ∘ wherein the determined auto-exposure settings of step b) are used. The method of the invention comprises a further step
ii) determining the concentration of the analyte in the sample by using the at least one image of at least part of the reagent test field of the optical test strip having the sample applied thereto, and at least part of the plurality of different color reference fields.

The method may comprise further steps which are not listed.

The term "auto-exposure settings" as used herein is a broad term and is to be given its ordinary and customary meaning to a person of ordinary skill in the art and is not to be limited to a special or customized meaning. The term specifically may refer, without limitation, to the automatically adjusted exposure parameter value(s) of a camera in order to control exposure or how much light is let in to an image. Exposure can, e.g., be regulated by adjusting exposure parameter values such as one or more of sensor gain, shutter speed or exposure time, lens aperture, or by adjusting any other operation of a camera for changing an exposure parameter value of the camera. Sensor gain may, e.g., be defined as a digital camera setting that controls the amplification of the signal from one or more of the camera sensors. Amplification of the signal may be controlled individually for each sensor or for a subset of sensors of the camera when adjusting sensor gain. Alternatively, amplification of the signal may be controlled uniformly for all sensors of the camera when adjusting sensor gain. Increasing the gain amplifies the signal by increasing the ratio of the analog-to-digital units (ADUs) to electrons acquired on the sensor. The result is that increasing gain increases the apparent brightness of an image. Shutter speed or exposure time may, e.g., be defined as the length of time the camera shutter is open, exposing light onto the camera sensor. Lens aperture may refer to a hole or an opening that limits the amount of light that can pass through. While for some mobile devices, exposure parameter values may be set manually by the user, many smartphones, especially cheaper ones, do not allow the user to freely adjust exposure parameter values like sensor gain, shutter speed (exposure time), or lens aperture. They only offer an automatic exposure mode. Such automatic exposure mode, e.g., may try to take the image such that the integral intensity or brightness of the image is in accordance with predefined values. However, when auto-exposure settings are determined based on an unsuitable scene in the set exposure metering area this may result in unsuitable auto-exposure settings for capturing an image and in consequence to an image unsuitable for determining the concentration of an analyte in the sample from such image.

The term "controlling" generally refers to a process of influencing or regulating settings or a process for determining settings in a defined or definable fashion. When auto-exposure settings are determined, the result (the determined auto-exposure settings) is influenced by the scene or part of the scene (or more specifically the light conditions in that scene or part of the scene) to be imaged. An exposure metering area can be set to determine which part of the scene (or the entire scene) in the field of view of the camera is used by the camera to determine the auto-exposure settings.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or a smartphone. Additionally or alternatively, as will be outlined in further detail below, the mobile device may also refer to a tablet computer or another type of portable computer having at least one camera.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. The camera may specifically comprise one or more imaging devices, such as camera chips or imaging chips, e.g. CCD and/or CMOS chips. The camera, in particular the imaging device, may comprise a one-dimensional or two-dimensional array of image sensors, such as pixels. As an example, the camera may comprise at least 10 pixels in at least one dimension, such as at least 10 pixels in each dimension. It shall be noted, however, that other cameras are also feasible. The camera, besides at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The camera may also comprise a diaphragm for controlling the aperture (whereby the amount of light that reaches the camera sensor may be controlled). The diaphragm functions much like the iris of the eye- it controls the effective diameter of the lens opening (called the aperture). The camera may further comprise an ambient light sensor for measuring light reflected by a scene to be captured in an image.

The invention specifically shall be applicable to cameras as usually used in mobile applications such as notebook computers, tablets or, specifically, cell phones such as smartphones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. A larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person. For example, gain can be a digital camera setting that controls the amplification of the signal from the camera sensor.

As outlined above, step a) comprises providing a color reference card and an optical test strip having a test field having a sample applied thereto.

The term "optical test strip" as used herein is a broad term and is to be given its ordinary and customary meaning to a person of ordinary skill in the art and is not to be limited to a special or customized meaning. The term specifically may refer, without limitation, to a strip shaped element or device configured for performing a color-change detection reaction. The optical test strip may also just be referred to as test strip herein, wherein these terms may refer to the same element. The optical test strip may particularly have a reagent test field containing at least one test chemical for detecting at least one analyte. The optical test strip, as an example, may comprise at least one substrate, such as at least one carrier, with the at least one reagent test field applied thereto or integrated therein. In particular, the optical test strip may further comprise at least one white area, such as a white field, specifically in a proximity to the test field, for example enclosing or surrounding the test field. The white area may be a separate field independently arranged on the substrate or carrier. However, additionally or alternatively, the substrate or carrier itself may be or may comprise the white area. The at least one carrier may be strip-shaped, thereby providing the basic form of the test strip. These test strips are generally widely in use and available. One test strip may carry a single test field or a plurality of test fields having identical or different test chemicals comprised therein.

As further used herein, the term "reagent test field", also referred to as "test field", is a broad term and is to be given its ordinary and customary meaning to a person of ordinary skill in the art and is not to be limited to a special or customized meaning. The term specifically may refer, without limitation, to a coherent amount of the test chemical, such as to a field or a region, e.g. a field of round, polygonal or rectangular shape, having one or more layers of material, with at least one layer of the test field having the test chemical comprised therein.

The optical test strip may be placed on top of the color reference card, and/or the color reference card may comprise one or more windows, wherein the color reference card, with the one or more windows, is placed on top of the optical test strip such that the reagent test region is visible through the window.

The term "color reference card" as used herein is a broad term and is to be given its ordinary and customary meaning to a person of ordinary skill in the art and is not to be limited to a special or customized meaning. The term specifically may refer, without limitation, to an arbitrary item having, disposed therein or disposed thereon, such as on at least one surface, a plurality of different color reference fields having known color properties or optical properties, such as having a plurality of colored fields having known reference color values, and further one or more gray background fields having defined gray levels. As an example, the color reference card may be a flat card comprising at least one substrate having, on at least one surface and/or disposed therein, the plurality of color reference fields having known color coordinates and one or more gray background fields having known gray levels. The color reference card may also comprise, one or more further gray fields. This will be described further below.

The substrate, specifically, may have a flat surface comprising the plurality of color reference fields, and the one or more gray background fields, and optionally (additional) gray fields disposed thereon (see below). The substrate, as an example, may be or may comprise one or more of a paper substrate, a cardboard substrate, a plastic substrate, a ceramic substrate or a metal substrate. Laminate substrates are also possible. The substrate, as an example, may be sheet-like or flexible. It shall be noted, however, that the substrate may also be implemented into an article of use, such as into a wall of a box, a vile, a container, a medical consumable, such as a test strip, or the like. Thus, the color reference card may also fully or partially be integrated into the optical test strip. Thus, the at least one image of at least a part of the color reference card may fully or partially comprise an image of at least part of the optical test strip having at least one reagent test field.

Further, the color reference card may comprise at least one position marker. The at least one position marker, as an example, may be or may comprise particularly an ArUco code or the like. Specifically, the at least one position marker may be arranged in at least one corner of the color reference card. Thus, the mobile device may be configured for detecting and/or reading the marker, specifically by optically detecting the marker (e.g., on the at least one image captured in step c)), and optionally retrieving information from the marker, such as information on the position and/or orientation of the color reference card in relation to the camera. The color reference card may include other marker(s) including further information or the position marker may additionally include further information. Such further information may include at least one of: an identifier for identifying the color reference card and/or the type of the color reference card, such as at least one of a label, a barcode or a QR-code; a specifier specifying details of the color reference card, such as reference color values, gray background and/or further gray values or the like, such as by using at least one of a label, a barcode or a QR-code.

The term "color reference field" as used herein is a broad term and is to be given its ordinary and customary meaning to a person of ordinary skill in the art and is not to be limited to a special or customized meaning. The term specifically may refer, without limitation, to an arbitrary item having known optical properties, such as a known reference color value. Specifically, a color reference field comprised by the color reference card may be a 2-dimensional structure, such as a rectangle, a square, a polygon, a circle and/or an ellipse, with a uniform color value. The color value of the color reference field specifically may be one or more of predetermined, known or determinable. The color reference field may be comprised by a surface of the color reference card and/or disposed therein, specifically in such a way that at least part of the plurality of different color reference fields (e.g. one color reference field) may be visible in the image captured in step c). Further, the color reference fields may have color values in a subspace of the color coordinate system corresponding to the color space of the color formation reaction of the reagent test region. The color reference fields of the color reference card specifically may be arranged in a regular pattern on the surface of the color reference card, such as in a rectangular pattern, e.g. a rectangular matrix pattern. The pattern arrangement specifically may enable identifying the color reference fields, such as by searching at a predetermined distance in an x- and/or y-direction from one or more of the position markers.

Further, the color reference fields may be locally distributed over the color reference card, specifically over a part of the color reference card visible in the image. The color reference card comprises at least two color reference fields having different reference color values. Specifically, the color reference card may comprise a plurality of color reference fields having different color values, wherein the color values of the color reference fields may be selected from a predetermined color subspace of the color space. The predetermined color subspace may comprise at least one color value of the color formation reaction of the reagent test field.

The term "known color reference values" as used herein is a broad term and is to be given its ordinary and customary meaning to a person of ordinary skill in the art and is not to be limited to a special or customized meaning. The term specifically may refer, without limitation, to a predetermined, real or true color value of a color reference field. Specifically, the known reference color value may comprise at least three color values, such as at least one color value for each R, G, B color. The known reference color value for each color reference field may be stored on a data storage device of the mobile device, for example by a look-up table, a register, a database or the like. The known reference color values may have been determined by measuring the respective color values, specifically by measuring the color values in a controlled laboratory environment, such as by using a photospectrometer. The measurement of the color reference fields using a photospectrometer may define the respective known reference color values.

The term "gray background field" as used herein is a broad term and is to be given its ordinary and customary meaning to a person of ordinary skill in the art and is not to be limited to a special or customized meaning. The term specifically may refer, without limitation, to an arbitrary item, such as a field having a known or determinable limitation, having a color or a gray shade of a defined gray level. Specifically, the gray level may refer to a relative brightness value indicating a proportion or percentage of a mixture of black and white. For example, a gray level of 100% may indicate a black field, a gray level of 0% may indicate a white field, whereas a gray level in between 0 and 100% may indicate a mixture of black and white. The one or more gray background fields comprised by the color reference card specifically may have defined and known RGB color values. Gray values result when r=g=b, for the color (r,g,b). The gray background field may be a 2-dimensional structure, such as a rectangle, a square, a polygon, a circle and/or an ellipse, with a uniform gray level. One gray background field may build an outer frame around an arrangement of the color reference fields (and optionally furthergray fields and positions markers). As another example, essentially all areas of the color reference card except for the color reference fields, and if present except for further gray fields and if present except for position markers (and potentially except for a space on the color reference card for placing the test strip) may be covered by the one or more gray background fields. Further, the one or more gray background fields comprised by the color reference card may be locally assigned to one or more of the test fields when the test strip is placed on top of the color reference card or beneath with some parts of the test strip being visible through a window of the color reference card. Further, the one or more gray background fields comprised by the color reference card may be locally assigned to the plurality of color reference fields and/or one or more further gray fields.

The one or more gray background fields may cover at least 5%, in particular at least 10% or at least 15% of the surface of the color reference card when facing the camera

As an example, the one or more gray background fields may have defined gray values between 0% and 30% gray level or between 70% and 100% gray level. By using gray background fields of gray levels at the lower or upper end of the gray spectrum the determination of the auto-exposure settings may be more strongly influenced than by using gray background fields having a gray level in the middle of the gray spectrum. Particularly, the one or more gray background fields may have defined gray values between 0% and 20% gray level or between 80% and 100% gray level. In one example, the one or more gray background fields may have defined gray values between 0% and 10% gray level. In one example the gray background fields are characterized by all having the same gray value.

In one example, the reference cared comprises further gray fields in addition to the one or more gray background fields. Such further gray fields may have gray values different from the one or more gray background fields. The further gray fields may, in particular, have gray levels between >30% and <70%. A further gray field may be a 2-dimensional structure, such as a rectangle, a square, a polygon, a circle and/or an ellipse, with a uniform gray level. In one example, the further gray fields each comprise a 2-dimensional structure smaller than the 2-dimensional structure of the biggest gray background field on the color reference card.

The further gray fields comprised by the color reference card may be locally assigned to one or more of the test fields when the test strip is placed on top of the color reference card or beneath with some parts of the test strip being visible through a window of the color reference card. The further gray fields comprised by the color reference card may be locally assigned to the plurality of color reference fields and/or one or more gray background fields.

The term "locally assigned" as used herein is a broad term and is to be given its ordinary and customary meaning to a person of ordinary skill in the art and is not to be limited to a special or customized meaning. The term specifically may refer, without limitation, to a predetermined spatial arrangement, such as a standardized local arrangement, between at least two objects. As an example, two or more objects may be locally assigned to each other by being placed in a neighboring position. Further gray fields may thus be placed in a neighboring position to the test field when the test strip is placed behind the color reference card. Further grey fields may also be locally assigned to the at least one color reference fields on the color reference card such that these further grey fields (at least partially) may surround the at least one color reference field. Thus, the term "locally" may refer to a nearest surrounding of the color reference field and/or the reagent test field. Therein, generally, if the first object is locally assigned to a second object, this assignment is not necessarily exclude the first object from also being locally assigned to at least one third object. As an example, the first object may be neighbored to at least one second object and at least one third object and, thereby, be assigned to both the second object and the third object.

The further gray fields may function as gray reference fields from which local brightness information may be determined. The one or more gray background fields may also function as gray reference fields from which local brightness information may be determined. Local brightness information may be taken into account for determining an intensity corrected image from the captured at least one image.

For example, further gray fields and optionally gray background fields may be locally assigned to the test field. They may thus surround the test field. The further gray fields and optionally the gray background fields locally assigned to the test field and the test field may thus form a test field group. The test field group may thus comprise a plurality of redundant further gray fields and optionally redundant gray background fields having at least two or optionally at least three different gray levels, wherein at least two, optionally at least three redundant gray fields may be provided for each gray level (counting the further gray fields and the gray background fields surrounding the test field here together as gray fields). Specifically, the redundant gray fields may be arranged around the test field in a symmetric fashion, more specifically in a rotationally symmetric and/or a mirror symmetric fashion. As an example, the local brightness information for the test field may be determined by averaging over redundant gray fields having the same gray level. Specifically, the local brightness information may be determined by averaging over redundant gray fields with respect to a distance of the gray fields to the test field. For example, the averaging may comprise weighting the color values of redundant gray fields with their reciprocal quadratic distance to the test field. Alternatively or additionally, the averaging may comprise averaging each of the RGB color values separately.

Similarly, the color reference card may comprise further gray fields and optionally gray background field locally assigned to the color reference fields. They may thus surround the color reference fields. The further gray fields and optionally the gray background fields locally assigned to a color reference field and the color reference field may thus form a color reference field group. The color reference field group may thus comprise a plurality of redundant further gray fields and optionally redundant gray background fields having at least two or optionally at least three different gray levels, wherein at least two optionally at least three redundant gray fields may be provided for each gray level (counting the further gray fields and the gray background fields surrounding the test field here together as gray fields). Specifically, the redundant gray fields may be arranged around the color reference field in a symmetric fashion, more specifically in a rotationally symmetric and/or a mirror symmetric fashion. As an example, the local brightness information for the color reference field may be determined by averaging over redundant gray fields having the same gray level. Specifically, the local brightness information may be determined by averaging over redundant gray fields with respect to a distance of the gray fields to the color reference field. For example, the averaging may comprise weighting the color values of redundant gray fields with their reciprocal quadratic distance to the color reference field. Alternatively or additionally, the averaging may comprise averaging each of the RGB color values separately.

As outlined above, step b) comprises setting an exposure metering area and determining auto-exposure settings based on the scene in the set exposure metering area.

The term "exposure metering area" as used herein is a broad term and is to be given its ordinary and customary meaning to a person of ordinary skill in the art and is not to be limited to a special or customized meaning. The term specifically may refer, without limitation, to the area that is considered by an auto-exposure algorithm to automatically calculate and/or manipulate exposure parameter value(s) of the camera in such a way that the image is captured in a predetermined manner. By the exposure metering area it can thus be determined which part of a scene in the field of view of the camera is considered for determining the auto-exposure settings. Determining auto-exposure settings can then be defined generally as running any algorithm that automatically calculates and/or manipulates one or more of the exposure parameter value of the camera in such a way that the image is captured in a predetermined manner. For example, there may be a predetermined optimum average brightness value for a given scene that the camera will try to achieve by adjusting one or more of the camera's exposure parameter values. Some auto-exposure algorithms, e.g., calculate and/or manipulate the exposure parameter values, e.g., the camera's gain, such that a mean, median, or weighted value of the image's brightness will equal a predetermined optimum brightness value.

The exposure metering area may be set to comprise the entire scene in the field of view of the camera or just a part of the scene in the field of view of the camera. In the first case, the scene in the set exposure metering area thus corresponds to the scene in the field of view of the camera. In the second case, the scene in the set exposure metering area only comprises part of the scene in the field of view of the camera. Determining auto-exposure settings based on the part of a scene in the set exposure metering area in the first case thus means to determine the auto-exposure settings based on the entire scene in the field of view of the camera (100 % part of the scene in the field of view of the camera). This may be also termed "whole-frame metering". According to the invention the exposure metering area is set to comprise just a part (e.g., at least 10% or at least 20% or at least 30%) of the scene in the field of view of the camera, which is a part in the center of the field of view of the camera (this may be termed "center spot metering"). In an alternative which does not form part of the claimed invention, the exposure metering area may be set to comprise a part of the scene in the field of the view of the camera that is off the center (this may be termed "off-center spot monitoring"), e.g., around an off-centered selected focus point. Determining auto-exposure settings based on the part of a scene in the set exposure metering area means to determine the auto-exposure settings based on that a part of the scene in the field of the view of the camera only. In one example, the scene of part of the scene in the field of view of the camera may comprise different zones which may be weighted differently for determining the auto-exposure settings. In another example, all zones of the scene or part of the scene are weighted evenly.

According to the method of the present invention the scene in the set exposure metering area comprises at least part of the reagent test field of the optical test strip having the sample applied thereto, and the entire color reference card.

Setting the exposure metering area and/or determining the auto-exposure settings may be initiated by user action or may automatically be initiated, e.g. once the presence of the at least one object within a field of view and/or within a predetermined part of the field of view of the camera is automatically detected. The user may set the exposure metering area manually, e.g., by pushing a button, or the processor of the mobile device may be configured, such as by software programming, for setting the exposure metering area. The exposure metering area may be set in a static or dynamic manner. For example, in a static setting, the exposure metering area may be set to cover a predefined part of the scene in the field of view of the camera, e.g. the part (area of a predefined size) in the center of the field of view of the camera. Static setting in particular refers to a setting of the exposure metering area without and/or before tracking the color card's position relative to the camera by the mobile device for determining the auto-exposure settings. The user may, e.g., be guided to place the color reference card in the center of the field of view of the camera after the exposure metering area is set to cover the center (an area of a predefined size) of the scene in the field of view of the camera. Dynamic setting in particular may refer to a setting of the exposure metering area wherein the color card's position relative to the camera by the mobile device is tracked first and, when the color card is determined to be suitably seen in (a certain part of) the field of view of the camera, the exposure metering area is set accordingly and auto-exposure settings are determined based on the scene in the set exposure metering area.

As outlined above, step c) comprises capturing, by using the camera at least one image comprising the scene of step b), wherein the determined auto-exposure settings of step b) are used.

The term "image" as used herein is a broad term and is to be given its ordinary and customary meaning to a person of ordinary skill in the art and is not to be limited to a special or customized meaning. The term may specifically refer, without limitation, to a set of spatially resolved optical data. Specifically, the term may relate to data recorded by using a camera, such as a plurality of electronic readings from the imaging device, such as the pixels of the camera chip.

The term "capturing at least one image" as used herein is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by user action or may automatically be initiated, e.g. once the presence of the at least one object within a field of view and/or within a predetermined part of the field of view of the camera is automatically detected. These automatic image acquisition techniques are known e.g. in the field of automatic barcode readers, such as from automatic barcode reading apps. The capturing of the images may take place, as an example, by acquiring a stream or "live stream" of images with the camera, wherein one or more of the images, automatically or by user interaction such as pushing a button, are stored and one of them is used as the at least one image. The image acquisition may be supported by a processor of the mobile device, and the storing of the images may take place in a data storage device of the mobile device.

The at least one image comprises the scene of step b). For capturing the image, the determined auto-exposure settings of step b) are used. As the brightness level of the color reference card, in particular the brightness level of the color reference card's gray background fields controls (within certain limits) which auto-exposure settings are determined in step b) and then used in step c) for capturing the at least one image, under- or overexposure of relevant parts on the captured image can be reduced or even prevented. This helps to obtain an image suitable for being used in the determining of the concentration of the analyte in the sample.

The mobile device may comprise a display. The camera and the display of the mobile device may both be positioned on the same side of the mobile device (in this case the camera may be termed a front camera) or on opposite sides of the mobile device (in this case the camera may be termed a back camera). Specifically, the camera may be a back camera. As one example, the mobile device may comprise a display, and the method of the present invention may comprise providing visual guidance on the display for positioning the camera relative to an object, the object comprising at least part of the optical test strip and/or at least part of the color reference card, for determining the auto-exposure settings and/or for capturing the at least one image. The visual guidance, e.g., comprises one or more arrows, frames or lines indicating a preferred positioning of the camera relative to the object. As one example, the visual guidance comprises an outline which corresponds to the shape of the object superimposed on the display of the mobile device.

Providing visual guidance on the display for positioning the camera relative to an object on the display for determining the exposure parameters and/or for capturing the at least one image may in particular relate to providing visual guidance on the display in order to guide the user to position the camera such that the scene in the set exposure metering area comprises at least part of the reagent test field of the optical test strip having the sample applied thereto, and at least part of the plurality of different color reference fields and at least part of the one or more gray background fields of the color reference card. The visual guidance may, e.g., comprise an outline which corresponds to the shape of at least part of the optical test strip and/or at least part of the color reference card superimposed on the display of the mobile device.

Determining the auto-exposure settings may be initiated automatically. The automatic determination of the auto-exposure settings may, e.g., be initiated automatically in case it is determined that the outline of the object superimposed on the display overlays the object. In particular, determining the auto-exposure settings may be initiated automatically once the presence and/or a certain position of the object within the set exposure metering area is automatically detected. The object may comprise at least part of the optical test strip and/or at least part of the color reference card. However, according to the invention, determining the auto-exposure settings is automatically initiated once the presence of at least one object within a predetermined part of the field of view of the camera is automatically detected, the object comprising the color reference card.

The color reference card may particularly comprise at least one position marker. Additionally or alternatively, the test strip may comprise at least one position marker. The at least one position marker, as an example, may be or may comprise an ArUco code or the like. Specifically, the at least one position marker may be arranged in at least one corner of the color reference card. As an example, position markers can also be located in each corner of the color reference card (and the color card potentially may comprise additional position markers).

The mobile device may be configured for detecting and/or reading the one or more position marker(s), specifically by optically detecting the marker(s). Thus, the mobile device may be configured for detecting and/or reading the marker(s) specifically by optically detecting the marker(s), and optionally retrieving information therefrom, such as information on the type, the properties or the position of the color reference card relative to the camera. Thereby, the mobile device may, e.g., automatically determine that the camera is in a defined position with respect to the color reference card. Determining the auto-exposure settings may be initiated automatically in case it is determined that the camera is in a defined position with respect to the color reference card based on the at least one position marker.

In one embodiment, the position of the camera with respect to the color reference card based on the at least one position marker is determined and the exposure metering area is set in dependence of the determined position. This is an example of dynamic setting of the exposure metering are. Specifically, the reference card's position relative to the camera can be tracked, e.g., by making use of the at least one position marker (such as at least one ArUco marker) and an appropriate exposure metering area can be set so that the scene in the exposure metering area comprises the color reference card for determining the auto-exposure settings.

The color reference card may contain at least one positioning element for positioning the optical test strip and/or the reagent test field. Specifically, the color reference card may comprise at least one window element through which the reagent test field is visible when the test strip is placed behind the color reference card. For example, the window element may be a cut-out portion of the color reference card. The window element may specifically be configured for keeping the optical test strip and, thus, the reagent test field comprised by the optical test strip, in the defined position with respect to the color reference card. The reagent test field may in particular be in a defined position with respect to the color reference card during one or both of determining the exposure parameters for the camera and capturing the at least one image. Thereby optimal performance, in terms of accuracy of the measurement results achieved may be ensured.

As described before, step c) of the method of the present invention comprises capturing, by using the camera, at least one image comprising the scene of step b) wherein the determined auto-exposure settings of step b) are used. During one or both of determining the exposure parameters for the camera and capturing the at least one image, the camera may be in a defined position with respect to the color reference card. In particular, the image may be captured with the camera and the color reference card being in the same relative position as for determining the auto-exposure settings.

The mobile device may automatically determine the presence of the color reference card and/or a certain position thereof within a field of view and/or within a predetermined part of the field of view of the camera, such as the set exposure metering area, based on the position marker(s). According to the invention, determining the auto-exposure settings is automatically initiated once the presence of at least one object within a predetermined part of the field of view of the camera is automatically detected, the object comprising the color reference card. Additionally, the capturing of the at least one image may be initiated automatically, in particular once the presence of at least one object within a field of view and/or within a predetermined part of the field of view of the camera is automatically detected, the object comprising at least part of the optical test strip and/or at least part of the color reference card.

Preferably, the light conditions during determining the exposure parameters for the camera and capturing the at least one image light conditions are substantially unchanged. Thereby, it may be ensured that the determined auto exposure settings are indeed suitable for capturing the image so that under- or overexposure of relevant parts on the captured image can be reduced or even prevented. In one embodiment, an illumination source of the mobile device, in particular the flash light of the mobile device, may be used to illuminate the scene in the field of view of the camera. Light conditions during determining the auto-exposure settings for the camera and capturing the image may thus be predominated by an illumination source of the mobile device, in particular the flash light of the mobile device.

At least 5%, in particular at least 10% or at least 15% of the scene in the set exposure metering area may consist of at least part of the one or more gray background fields. In one example, the scene in the set exposure metering area may comprise the entire color reference card. The at least one image captured in step c) may essentially consist of the scene in the set exposure metering of step b). One or both of this may contribute to influencing auto-exposure settings by the gray background fields having a defined gray value such that over and/or underexposure of the image captured in step c) is avoided or at least reduced. In one embodiment, auto-exposure settings may thus be influenced in a way that brightness of a least one color reference field is different from a situation wherein the gray background fields having a defined gray value would be replaced by white fields. In another embodiment, auto-exposure settings may thus be influenced in a way that brightness of a least one color reference field is different from a situation wherein the gray background fields having a defined gray value would be replaced by black fields.

As step d) the method may comprise determining at least one item of admissibility information, wherein the item of admissibility information indicates admissibility in case each pixel or at least a predetermined group of pixels corresponding to the color reference fields in the at least one image exhibit brightness values within acceptable limits, for instance between 50 and 240, or between 80 and 220. The predetermined group of pixels may be dynamically determined in the field of view of the camera based on the at least one position marker. The term "admissibility" as used herein is a broad term and is to be given its ordinary and customary meaning to a person of ordinary skill in the art and is not to be limited to a special or customized meaning. The term specifically may refer, without limitation, to a characterization whether the captured at least one image is permitted and/or denied for being used in the determination of the concentration of the analyte. Thus, as an example, the admissibility may be qualified or quantified by determining whether each pixel or at least a predetermined group of pixels corresponding to the color reference fields in the at least one image exhibit brightness values within acceptable limits. The brightness values may be compared with one or more conditions. As a simple example, the brightness values may be compared with one or more comparative values, reference values or standard values, wherein the comparison may result in a binary result such as "admissible" or "not admissible"/"inadmissible". As an example, the comparative and/or reference values may comprise a minimum allowable brightness value and a maximum allowable brightness value. The comparative values, reference values and/or standard values may be derived, as an example, from experiments or from boundary conditions determined e.g. by a precision to be achieved in the analytical measurement.

The term "item of admissibility information" as used herein is a broad term and is to be given its ordinary and customary meaning to a person of ordinary skill in the art and is not to be limited to a special or customized meaning. The term specifically may refer, without limitation, to an indication of information regarding the admissibility. In particular, the item of admissibility information may refer to an indication of the admissibility of determining an analytical measurement result value from the at least one image. The item of admissibility information, as an example, may be Boolean or digital information, such as indicating "admissible" or "not admissible"/"inadmissible". Thus, as an example, in case the brightness value is below the minimum brightness value or above the maximum brightness value the captured image may be determined as being inadmissible for the purpose of determining an analytical measurement result value.

In a further aspect not forming part of the claimed invention, a method for determining a concentration of an analyte in a bodily fluid is disclosed. The method comprises using a mobile device having at least one camera.

The method further comprises:
i) controlling auto-exposure settings of the mobile device according to the present invention, such as according to any of the embodiments disclosed above and/or according to any one of the embodiments disclosed in further detail below;
ii) determining the concentration of the analyte in the sample by using the at least one image of at least part of the reagent test field of the optical test strip having the sample applied thereto, and at least part of the plurality of different color reference fields.

The term "determining the concentration of an analyte in a bodily fluid", also referred to as an "analytical measurement", as used herein is a broad term and is to be given its ordinary and customary meaning to a person of ordinary skill in the art and is not to be limited to a special or customized meaning. The term specifically may refer, without limitation, to a quantitatively and/or qualitatively determination of at least one analyte in an arbitrary sample or aliquot of bodily fluid. For example, the bodily fluid may comprise one or more of blood, interstitial fluid, urine, saliva or other types of body fluids. In one embodiment, the analyte is glucose and the bodily fluid is blood. The result of the determining of the concentration, as an example, may be a concentration of the analyte and/or the presence or absence of the analyte to be determined. Specifically, as an example, the determination may be a blood glucose measurement, thus the result of the determination may for example be a blood glucose concentration. In particular, an analytical measurement result value may be determined by the analytical measurement.

Consequently, the term "analyte concentration value", often also referred to as "analytical measurement result value", as used herein, is a broad term and is to be given its ordinary and customary meaning to a person of ordinary skill in the art and is not to be limited to a special or customized meaning. The term specifically may refer, without limitation, to a numerical indication of an analyte concentration in a sample.

The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value.

The method for determining a concentration of an analyte in a bodily fluid further comprises using the mobile device having the at least one camera. Further, the method comprises using a color reference card and an optical test strip having a test field having a sample applied thereto.

The method comprises determining the analyte concentration value based on a color formation of the reagent test field. Thus, the method may be an analytical measurement including inducing a test reaction between the test chemical and a sample of the bodily fluid or a part thereof, such as the at least one analyte, specifically an analyte-specific test reaction, wherein the test reaction includes a color change of the reagent test field indicative of a degree of the test reaction and/or indicative of a presence or a concentration of the analyte. The color formation may include an arbitrary change of at least one optical property of the optical test strip or, specifically, of the reagent test field, which change may be measured or determined optically by using the camera. Specifically, the analytical measurement may be or may comprise a color formation reaction in the presence of the at least one analyte to be determined. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction. The color formation may be detected by the mobile device, such as by a processor of the mobile device, and may be evaluated quantitatively, such as by deriving, from the at least one image, at least one parameter quantifying or characterizing the color formation of the reagent test field due to the presence of the analyte in the sample of the bodily fluid. As an example, one or more of the above-mentioned color coordinates may be used. Thus, the mobile device and specifically the processor of the mobile device may be configured for determining a color change by determining a change of one or more color coordinates taking place due to the detection reaction.

The at least one analyte concentration value is determined from the color formation of the reagent test field. For this purpose, the at least one image may be used. The analyte concentration value, as an example, may be a numerical value indicator of a result of the analytical measurement, such as indicative of the concentration of at least one analyte in the sample, such as a blood glucose concentration.

As an example, when capturing the at least one image of at least part of the reagent test field, at least part of the plurality of different color reference fields and at least part of the one or more gray background fields of the color reference card are in the field of view of the camera and, thus, at least a part of the color reference card may be visible in the at least one image of the at least one part of the reagent test field. As an example, the optical test strip may be placed on top of the color reference card, and/or the color reference card may comprise one or more windows (cut-outs), wherein the color reference card, with the one or more windows, is placed on top of the optical test strip such that the reagent test field is visible through the window.

The use of the color reference card specifically may allow for correcting camera specific or device specific changes in the at least one image of the color of the reagent test region. Thus, typically, cameras and/or mobile devices, without notifying the user, apply one or more evaluation or pre-evaluation algorithms to the image, such as gamma corrections, which have to be taken into account when evaluating the images and determining the at least one analyte concentration value. By using the at least one color reference card having known optical properties, the mobile device may be set up for calibrating and/or correcting the image, thus taking into account the internal processes of the camera and/or the mobile device when or before determining the at least one analyte concentration value. As outlined above, the method for determining the concentration of an analyte in a bodily fluid comprises the method of controlling auto-exposure settings.

In a further aspect not forming part of the claimed invention, a mobile device is disclosed, the mobile device having at least one camera and at least one display. The mobile device further has at least one processor. The mobile device is configured for performing at least steps b) and c) and optionally step d) of the method of controlling auto-exposure settings according to the present invention, such as according to any one of the embodiments disclosed above and/or according to any one of the embodiments disclosed in further detail below.

The mobile device may further be configured for performing step ii) of the method of determining the concentration of an analyte in a sample according to the present invention, such as according to any one of the embodiments disclosed above and/or according to any one of the embodiments disclosed in further detail below.

The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the mobile device, computer or system. As an example, the processor may comprise at least one arithmetic logic unit (ALU), at least one floating-point unit (FPU), such as a math co-processor or a numeric coprocessor, a plurality of registers, specifically registers configured for supplying operands to the ALU and storing results of operations, and a memory, such as an L1 and L2 cache memory. In particular, the processor may be a multicore processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like.

The processor may specifically be configured, such as by software programming for performing and/or supporting one or more of the method steps of the method of controlling auto-exposure settings of the mobile device. Specifically, the processor may be configured for supporting the setting an exposure metering area and determining auto-exposure settings based on the scene in the set exposure metering area and for the capturing of the image, such as by prompting the user to capture the image and/or by detecting the color reference card in a field of view, and automatically setting an exposure metering area and determining auto-exposure settings based on the scene in the set exposure metering area and/or automatically capturing the image. Further, the processor may be configured for identifying color reference fields on the captured image and for determining measured reference color values thereof. The processor may be configured for assigning known reference color values to the corresponding measured color reference fields. Thus, the processor may be configured for determining a relationship between measured reference color values and corresponding known reference color values, for example by fitting the measured reference color values to the corresponding known reference color values. The processor may determine the relationship, for example the linear transformation such as the color transformation matrix, using a linear optimization approach. Further, the processor may be configured for deriving the at least one item of quality information on the quality of the color reference card by using the relationship.

Further, the processor may be configured, such as by software programming, for performing and/or supporting the method steps of the method for determining the concentration of an analyte in a bodily fluid. Specifically, the processor may be configured for supporting the capturing of the at least one image comprising at least part of the reagent test field of the optical test strip having the sample applied thereto, and at least part of the plurality of different color reference fields and at least part of the one or more gray background fields by using the camera of the mobile device. The processor may further be configured for determining at least one analyte concentration value from color formation of the reagent test region, such as by controlling auto-exposure settings and deriving the change of one or more color coordinates taking place due to the color formation reaction and transforming the change of one or more color coordinates into the at least one analyte concentration value. The processor specifically may be configured for supporting one or more or all of steps b), c) and d) of the method for controlling auto-exposure settings and step ii) of the method - of determining the concentration of an analyte in a bodily fluid. The processor may further be configured for guiding the user in providing a color reference card and an optical test strip having a test field having a sample applied thereto, such as by providing user guidance, e.g. in a visual format or in an audible format. The processor may further be configured for supporting the capturing of the at least one image, e.g. by automatically detecting the optical test strip or a part thereof in a field of view and/or by prompting the user to capture the image.

In a further aspect not forming part of the claimed invention, a kit is disclosed, the kit comprising at least one mobile device according to the present disclosure, such as according to any one of the embodiments disclosed above and/or according to any one of the embodiments disclosed in further detail below, further comprising at least one optical test strip having at least one reagent test field, and at least one color reference card comprising a plurality of different color reference fields having known reference color values and one or more gray background fields having a defined gray value. The term "kit" as used herein is a broad term and is to be given its ordinary and customary meaning to a person of ordinary skill in the art and is not to be limited to a special or customized meaning. The term specifically may refer, without limitation, to a combination of at least two items which might as an example, may be supplied conjointly in a package, which may interact in order to fulfill at least one common purpose.

In a further aspect not forming part of the claimed invention, a computer program is disclosed, the computer program comprising instructions which, when the program is executed by a mobile device having a camera cause the processor of the mobile device to perform steps b) and c) (and optionally also step d) of the method for controlling auto-exposure settings, and/or step ii) of the method of determining the concentration of an analyte in a bodily fluid according to any one of the embodiments disclosed above and/or according to any one of the embodiments disclosed in further detail below.

In a further aspect not forming part of the claimed invention, a computer-readable storage medium is disclosed, the computer-readable storage medium comprising instructions which, when the program is executed by a mobile device having a camera cause the processor of the mobile device to perform steps b) and c) (and optionally also step d) of the method for controlling auto-exposure settings, and/or step ii) of the method of determining the concentration of an analyte in a bodily fluid according to any one of the embodiments disclosed above and/or according to any one of the embodiments disclosed in further detail below.

As used herein, the term "computer-readable storage medium" specifically may refer to a non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

The computer program may also be embodied as a computer program product. As used herein, a computer program product may refer to the program as a tradable product. The product may generally exist in an arbitrary format, such as in a paper format, or on a computer-readable data carrier and/or on a computer-readable storage medium. Specifically, the computer program product may be distributed over a data network.

The methods and devices according to the present invention enable the use of a greater variety of models of mobile devices for the analytical measurement. By using the quality checked color reference card, the analytical measurement may provide means for an accurate analytical measurement of the analyte concentration in a bodily fluid even for a great variety of different mobile devices.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
Figure 1 shows a color reference card in plan view;
Figure 2 shows an embodiment of a kit comprising the mobile device, a color reference card and an optical test strip;
Figure 3 shows the situation when determining auto-exposure settings based on the scene in the set exposure metering area and capturing the image;
Figure 4 shows a flow chart of an embodiment of a method of controlling auto-exposure settings; and
Figure 5 shows a flow chart of an embodiment of a method for determining the concentration of at least one analyte in a bodily fluid.

### Detailed description of the embodiments

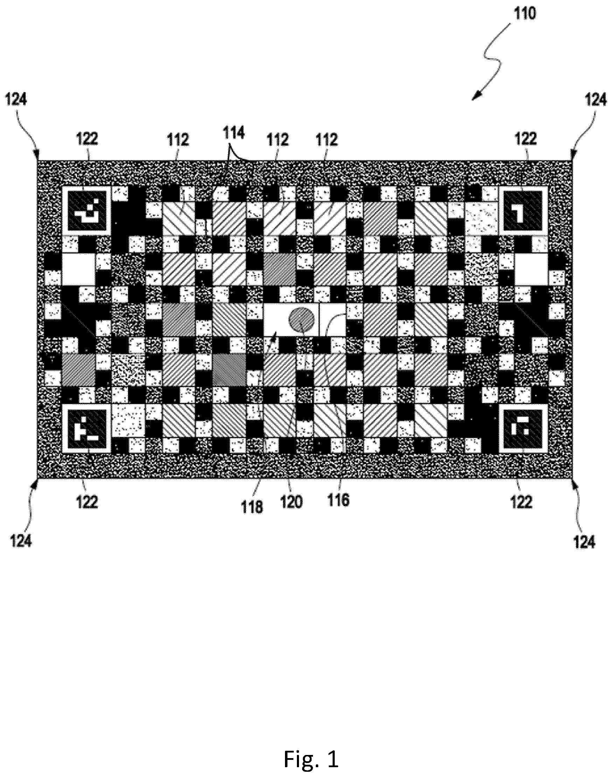

In Figure 1, an exemplary embodiment of a color reference card 110 is shown in a plan view. The color reference card 110 comprises a plurality of color reference fields 112 having known reference color values. The color reference fields 112 may be arranged on the surface of the color reference card 110, such as on a substrate of the color reference card 110. In particular, the color reference fields 112 may be distributed equally over the surface of the color reference card 110, specifically in such a way that the plurality of color reference fields 112 may be distributed over the entire surface of the color reference card 110. As an example, the color reference fields 112 may be arranged in matrix pattern, such as a rectangular matrix pattern. However, the color reference fields 112 may also be arranged in other ways, such as separately from each other. The color reference card 110 further comprises one or more gray background fields 114 surrounding the color reference fields 112 and/or framing the color reference card. The color reference fields 112 and the gray background fields 114 may not overlap each other.

The color reference card 110 may further comprise at least one window 116. Thus at least one optical test strip 118 or a part thereof may be visible through the window 116 when the color reference card 110 is placed on top of the optical test strip 118. Specifically, at least one reagent test field 120 comprised by the optical test strip 118 may be visible through the window 116 of the color reference card 110. As another example, the color reference card 110 may comprise the optical test strip 118 having at least one reagent test field 120, specifically in such a way that the at least one reagent test field 120 is accessible and visible.

Further, the color reference card 110 may comprise at least one position marker 122. The position marker 122 may particularly be an ArUco code. In Figure 1, the position marker 122 specifically may comprise one or more ArUco codes, such as in the corners of a rectangular matrix comprising the color reference fields 112. Thus, generally, the position marker 122 may be arranged in at least one corner 124 of the color reference card 110. For example, at least one position marker 122 may be arranged in each of the corners 124 of the color reference card 110, specifically in such a way that the position marker 122 may be visible together with the plurality of color reference fields 112. Further, the position marker 122 may comprise information about the orientation of the color reference card 110.

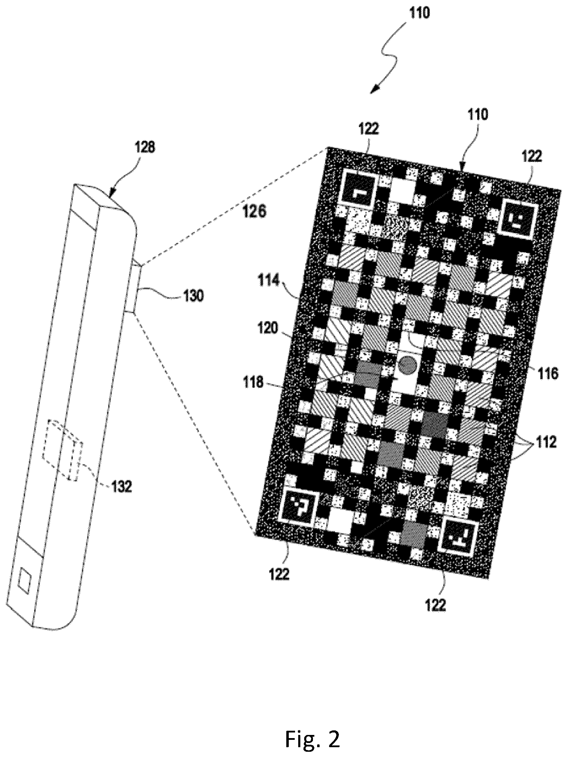

In Figure 2, an exemplary embodiment of a kit is shown in a perspective view. The kit comprises at least one mobile device 128 and at least one color reference card 110. Further, the kit comprises the at least one optical test strip 118.

The mobile device 128 may be or may comprise at least one of a cell phone, a smartphone, a tablet computer or the like. Further, the mobile device 128 has at least one camera 130. The camera 130 of the mobile device 128 may be configured for recording images, specifically color images. Thus, the camera 130 may be a color camera and may comprise at least three color sensors, such as at least one color sensor for the R, G, B colors.

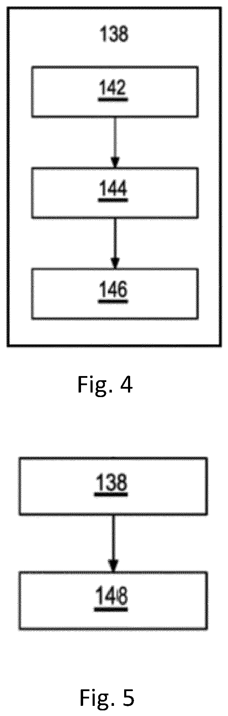

Further, the mobile device 128 may comprise at least one processor 132. The processor132 may be configured, specifically by software programming, to perform one or more of the method steps b), c) and d) of the method of controlling auto-exposure settings. Further, the processor 132 may be configured for supporting step ii) of the method for determining a concentration of an analyte. Exemplary embodiments of the above-mentioned methods are shown in Figures 4 and 5, respectively, and will be described in further detail below. Thus, reference may be made to the description of Figures 4 and 5.

The processor 132 may specifically be configured for supporting the setting of an exposure metering area and determining auto-exposure settings based on the scene 126 in the set exposure metering area. In the embodiment shown, the scene 126 in the set exposure metering area comprises the color reference card and the reagent test field. The processor 132 may further specifically be configured for capturing of at least one image comprising the scene 126. Specifically, the processor 132 may prompt a user of the mobile device 128 to capture the image. Additionally or alternatively, the processor 132 may be configured for automatically capturing the image of the color reference card 110, specifically when the color reference card 110 may be in a field of view.

The color reference card 110 has been described above (see Figure 1). The color reference card 110 comprises the plurality of color reference fields 112 having known reference color values. Further, the color reference card 110 may comprise the at least one window 116. Thus, the optical test strip 118 may be visible through the window 116 of the color reference card 110, specifically when the color reference card 110 may be placed on top of the optical test strip 118 such that both, the color reference card 110 and the optical test strip 118, may be visible on the at least one image captured by the camera 130 of the mobile device 128. Specifically, the at least one reagent test region 120 of the optical test strip 118 may be visible through the window 116 of the color reference card 110.

The color reference card 110 may further comprise the at least one position marker 122. The position marker 122 may be arranged on at least one surface of the color reference card 110 such that the marker 122 may be detectable by the camera 130 of the mobile device 128. The at least one marker 122 may be used for identifying the color reference card 110. Specifically, the processor 132 of the mobile device 128 may be configured for detecting the position marker 122 in a field of view of the camera 130.

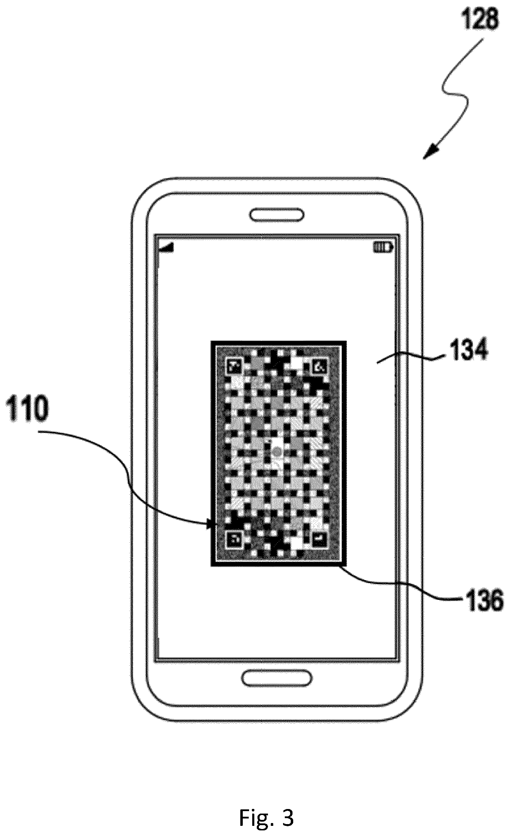

Figure 3 shows the situation when determining auto-exposure settings and capturing the image. Visual guidance is provided in this embodiment on the display in form of an outline 136 of the color reference card superimposed on the display 134 of the mobile device 128 in order to guide the user to position the camera such that the scene in the set exposure metering area which corresponds to the outline 136 comprises at least part of the reagent test field of the optical test strip having the sample applied thereto, and at least part of the plurality of different color reference fields and at least part of the one or more gray background fields. In the example shown, the scene in the set exposure metering area comprises the entire color reference card.

Determining the auto-exposure settings and capturing the image may be initiated automatically. The automatic determination of the auto-exposure settings may, e.g., be initiated automatically in case it is detected that, based on the position marker 122, the color reference card is within the set exposure metering area. Similarly, the image can then be automatically captured wherein the determined auto-exposure settings are used.

Figure 4 shows a flow chart of an exemplary embodiment of a method of controlling auto-exposure settings of a mobile device 138.

The method comprises:
a) (denoted with reference number 142) providing a color reference card and an optical test strip having a test field having a sample applied thereto,
   - wherein the color reference card comprises a plurality of different color reference fields having known reference color values and one or more gray background fields having defined gray values,
b) (denoted with reference number 144) setting an exposure metering area and determining auto-exposure settings based on the scene in the set exposure metering area;
   - wherein the scene in the set exposure metering comprises at least part of the reagent test field of the optical test strip having the sample applied thereto, and at least part of the plurality of different color reference fields and at least part of the one or more gray background fields of the color reference card, and
c) (denoted with reference number 146) capturing, by using the camera, an image comprising the scene of step b)
   - wherein the determined auto-exposure settings of step b) are used.

The method may comprise additional steps that are not listed.

In step a) a color reference card 110 and an optical test strip 118 having a test field having a sample applied are provided. The processor 132 may be configured for guiding the user in providing the color reference card and the optical test strip by providing user guidance, e.g. in a visual format or in an audible format

In step b) the exposure metering area is set and auto-exposure settings based on the scene 126 in the set exposure metering area are determined. For example, the processor 132 may be configured for prompting the user to place the color reference card in the field of view of the camera or the position of the color reference card 110 may be tracked by the mobile device via the position marker 122. As an example, the processor 132 may be configured for detecting the color reference card 110 in a field of view and further for determining the auto-exposure settings when the color reference card with the position marker 122 is detected in the set exposure metering area.

In step c) an image is captured. The capturing of the at least one image may be initiated by user action or may automatically be initiated, e.g. once the color reference card is detected in the field of view of the camera 130 based on the position markers 122. The image acquisition may be supported by processor 132 of the mobile device 128, and the storing of the images may take place in a data storage device of the mobile device. The at least one image comprises the scene of step b). For capturing the image, the determined auto-exposure settings of step b) are used.

In an experiment, two color reference cards similar to the one shown in Figure 1 with gray background fields of different gray values were compared. Auto-exposure settings were determined based on each color reference card placed in the exposure metering area and images of each color reference card were then captured with the determined auto-exposure settings, respectively.

The following table shows how the brightness level of the gray background fields influenced the detected brightness of an exemplary color reference field on the color reference card by influencing the auto-exposure settings.

| Gray level of gray background field | Brightness of color reference field (r,g,b) |
|---|---|
| Lighter gray | (138/155/36) average of 20 images |
| Darker gray | (148/168/40) average of 13 images |

The brightness of the exemplary color reference field was lower when a color reference card with brighter gray background fields was used compared to a color reference card with darker gray background fields. Even brighter or even darker gray background fields may be used to steer exposure levels more strongly to a desired exposure level. This can help to obtain images suitable for being used in the determining of the concentration of an analyte in a sample applied to the test field of a test strip when imaged together with the color reference card (e.g. by avoiding over- or underexposure of the test field or relevant fields of the color reference card needed for image correction in determining the analyte concentration).

Figure 5 shows a flow chart of an exemplary embodiment of a method of determining the concentration of an analyte in a sample by using a mobile device 128 having at least one camera 130.

The method comprises:
i) (denoted with reference number 138) controlling auto-exposure settings of the mobile device as described before when capturing, by using the camera (130), at least one image of at least part of a color reference card (110) and of at least part of a reagent test field (120) of an optical test strip (118) having a sample applied thereto,
ii) (denoted with reference number 148) determining the concentration of the analyte in the sample by using the at least one image of at least part of the reagent test field (120) of the optical test strip (118) having the sample applied thereto, and at least part of the plurality of different color reference fields (112).

Regard step i) it is referred to the method of controlling auto-exposure settings 138 as described above. In step ii), e.g., the glucose concentration in a blood sample applied to the test field of the test strip may be determined from the color formation of the reagent test field using the at least one image captured. By using intensity values determined from one or more color reference fields card having known optical properties, the mobile device may be set up for calibrating and/or correcting the image, thus taking into account the internal processes of the camera and/or the mobile device when or before determining the at least one analyte concentration value.

### List of reference numbers

- 110: color reference card
- 112: color reference field
- 114: gray background field
- 116: window
- 118: optical test strip
- 120: reagent test field
- 122: position marker
- 124: corner
- 126: scene
- 128: mobile device
- 130: camera
- 132: processor
- 134: display
- 136: outline
- 138: controlling auto-exposure settings of a mobile device
- 140: providing a color reference card (110) and an optical test strip
- 142: setting an exposure metering area and determining auto-exposure settings
- 144: capturing an image
- 148: determining the concentration of an analyte

## Claims

1. A method of determining the concentration of an analyte in a sample by using a mobile device (128) having at least one camera (130), the method comprising the following steps:
i) controlling auto-exposure settings of the mobile device (128) when capturing, by using the camera (130), at least one image of at least part of a color reference card (110) and of at least part of a reagent test field (120) of an optical test strip (118) having a sample applied thereto, comprising
a) providing a color reference card (110) and an optical test strip (118) having a test field (120) having a sample applied thereto,
∘ wherein the color reference card (110) comprises a plurality of different color reference fields (112) having known reference color values and one or more gray background fields (114) having defined gray values,
b) setting an exposure metering area and determining auto-exposure settings based on the scene (126) in the set exposure metering area, wherein the exposure metering area is set to comprise just a part in the center of the field of view of the camera,
wherein the scene (126) in the set exposure metering area comprises at least part of the reagent test field (120) of the optical test strip (118) having the sample applied thereto, and the entire the color reference card (110), and wherein determining the auto-exposure settings is automatically initiated once the presence of at least one object within a predetermined part of the field of view of the camera is automatically detected, the object comprising the color reference card
c) capturing, by using the camera (120), at least one image comprising the scene (126) of step b),
∘ wherein the determined auto-exposure settings of step b) are used
ii) determining the concentration of the analyte in the sample by using the at least one image of at least part of the reagent test field (120) of the optical test strip (118) having the sample applied thereto, and at least part of the plurality of different color reference fields (112).

2. The method according to any of the preceding claims, wherein the mobile device (128) comprises a display (134), and the method further comprises in step i) the step of:
∘ providing visual guidance on the display (134) for positioning the camera (130) relative to an object, the object comprising at least part of the optical test strip (118) and/or at least part of the color reference card (110), for determining the auto-exposure settings and/or for capturing the at least one image.

3. The method according to any of the preceding claims, wherein the color reference card and/or the test strip comprises at least one position marker (122).

4. The method according to the preceding claim, wherein determining the auto-exposure settings is initiated automatically in case it is determined that the camera (130) is in a defined position with respect to the color reference card (110) based on the at least one position marker (122).

5. The method according to any of the two preceding claims, wherein the position of the camera (130) with respect to the color reference card (110) based on the at least one position marker (122) is determined and the exposure metering area is set in dependence of the determined position.

6. The method according to any of the preceding claims, wherein the reagent test field (120) is in a defined position with respect to the color reference card (110) during one or both of determining the auto-exposure settings for the camera (130) and capturing the at least one image.

7. The method according to any of the preceding claims, wherein at least 5%, in particular at least 10% or at least 15% of the scene in the set exposure metering area consists of at least part of the one or more gray background fields (114).

8. The method according to any of the preceding claims, wherein the method in step i) further comprises:
d) determining at least one item of admissibility information, wherein the item of admissibility information indicates admissibility in case each pixel or at least a predetermined group of pixels corresponding to the color reference fields (112) in the at least one image exhibit brightness values within acceptable limits, for instance between 50 and 240, or between 80 and 220.

## Patentansprüche

1. Verfahren zum Bestimmen der Konzentration eines Analyten in einer Probe unter Verwendung einer mobilen Vorrichtung (128) mit mindestens einer Kamera (130), wobei das Verfahren die folgenden Schritte umfasst:
i) Steuern von automatischen Belichtungseinstellungen der mobilen Vorrichtung (128), wenn unter Verwendung der Kamera (130) mindestens ein Bild von mindestens einem Teil einer Farbreferenzkarte (110) und von mindestens einem Teil eines Reagenztestfelds (120) eines optischen Teststreifens (118) mit einer darauf aufgebrachten Probe aufgenommen wird, umfassend
a) Bereitstellen einer Farbreferenzkarte (110) und eines optischen Teststreifens (118) mit einem Testfeld (120) mit einer darauf aufgebrachten Probe,
○ wobei die Farbreferenzkarte (110) eine Vielzahl von verschiedenen Farbreferenzfeldern (112) mit bekannten Referenzfarbwerten und ein oder mehrere Grauhintergrundfelder (114) mit definierten Grauwerten umfasst,
b) Einstellen eines Belichtungsmessbereichs und Bestimmen von automatischen Belichtungseinstellungen basierend auf der Szene (126) in dem eingestellten Belichtungsmessbereich, wobei der Belichtungsmessbereich so eingestellt ist, dass er nur einen Teil in der Mitte des Sichtfelds der Kamera umfasst,
wobei die Szene (126) in dem eingestellten Belichtungsmessbereich mindestens einen Teil des Reagenztestfelds (120) des optischen Teststreifens (118) mit der darauf aufgebrachten Probe und die gesamte Farbreferenzkarte (110) umfasst und wobei das Bestimmen der automatischen Belichtungseinstellungen automatisch eingeleitet wird, sobald das Vorhandensein von mindestens einem Objekt innerhalb eines vorbestimmten Teils des Sichtfelds der Kamera automatisch detektiert wird, wobei das Objekt die Farbreferenzkarte umfasst,
c) Aufnehmen mindestens eines Bilds, das die Szene (126) von Schritt b) umfasst, unter Verwendung der Kamera (120),
○ wobei die bestimmten automatischen Belichtungseinstellungen von Schritt b) verwendet werden,
ii) Bestimmen der Konzentration des Analyten in der Probe unter Verwendung des mindestens einen Bilds von mindestens einem Teil des Reagenztestfelds (120) des optischen Teststreifens (118) mit der darauf aufgebrachten Probe und mindestens einem Teil der Vielzahl von verschiedenen Farbreferenzfeldern (112).

2. Verfahren nach einem der vorhergehenden Ansprüche, wobei die mobile Vorrichtung (128) eine Anzeige (134) umfasst und das Verfahren ferner in Schritt i) den folgenden Schritt umfasst:
○ Bereitstellen einer visuellen Führung auf der Anzeige (134) zum Positionieren der Kamera (130) relativ zu einem Objekt, wobei das Objekt mindestens einen Teil des optischen Teststreifens (118) und/oder mindestens einen Teil der Farbreferenzkarte (110) umfasst, zum Bestimmen der automatischen Belichtungseinstellungen und/oder zum Aufnehmen des mindestens einen Bilds.

3. Verfahren nach einem der vorhergehenden Ansprüche, wobei die Farbreferenzkarte und/oder der Teststreifen mindestens eine Positionsmarkierung (122) umfasst.

4. Verfahren nach dem vorhergehenden Anspruch, wobei das Bestimmen der automatischen Belichtungseinstellungen automatisch eingeleitet wird, falls basierend auf der mindestens einen Positionsmarkierung (122) bestimmt wird, dass sich die Kamera (130) in einer definierten Position in Bezug auf die Farbreferenzkarte (110) befindet.

5. Verfahren nach einem der zwei vorhergehenden Ansprüche, wobei die Position der Kamera (130) in Bezug auf die Farbreferenzkarte (110) basierend auf der mindestens einen Positionsmarkierung (122) bestimmt wird und der Belichtungsmessbereich in Abhängigkeit von der bestimmten Position eingestellt wird.

6. Verfahren nach einem der vorhergehenden Ansprüche, wobei sich das Reagenztestfeld (120) während eines oder beider des Bestimmens der automatischen Belichtungseinstellungen für die Kamera (130) und des Aufnehmens des mindestens einen Bilds in einer definierten Position in Bezug auf die Farbreferenzkarte (110) befindet.

7. Verfahren nach einem der vorhergehenden Ansprüche, wobei mindestens 5 %, insbesondere mindestens 10 % oder mindestens 15 % der Szene in dem eingestellten Belichtungsmessbereich aus mindestens einem Teil des einen oder der mehreren Grauhintergrundfelder (114) bestehen.

8. Verfahren nach einem der vorhergehenden Ansprüche, wobei das Verfahren in Schritt i) ferner Folgendes umfasst:
d) Bestimmen von mindestens einer Zulässigkeitsinformation, wobei die Zulässigkeitsinformation die Zulässigkeit angibt, falls jedes Pixel oder mindestens eine vorbestimmte Gruppe von Pixeln, die den Farbreferenzfeldern (112) in dem mindestens einen Bild entsprechen, Helligkeitswerte innerhalb akzeptabler Grenzen aufweisen, zum Beispiel zwischen 50 und 240 oder zwischen 80 und 220.

## Revendications

1. Procédé de détermination de la concentration d'un analyte dans un échantillon en utilisant un dispositif mobile (128) doté d'au moins une caméra (130), le procédé comprenant les étapes suivantes :
i) commande des réglages d'exposition automatique du dispositif mobile (128) lors de la capture, en utilisant la caméra (130), d'au moins une image d'au moins une partie d'une carte de référence de couleurs (110) et d'au moins une partie d'un champ de test de réactif (120) d'une bandelette de test optique (118) ayant un échantillon appliqué sur celle-ci, comprenant
a) la fourniture d'une carte de référence de couleurs (110) et d'une bandelette de test optique (118) dotée d'un champ de test (120) ayant un échantillon appliqué sur celui-ci,
○ dans lequel la carte de référence de couleurs (110) comprend une pluralité de champs de référence de couleurs (112) différents ayant des valeurs de couleur de référence connues et un ou plusieurs champs d'arrière-plan de gris (114) ayant des valeurs de gris définies,
b) le réglage d'une zone de mesure d'exposition et la détermination de réglages d'exposition automatique en se basant sur la scène (126) dans la zone de mesure d'exposition réglée, dans lequel la zone de mesure d'exposition est réglée pour comprendre seulement une partie au centre du champ de vision de la caméra,
dans lequel la scène (126) dans la zone de mesure d'exposition réglée comprend au moins une partie du champ de test de réactif (120) de la bandelette de test optique (118) ayant l'échantillon appliqué sur celle-ci, et la totalité de la carte de référence de couleurs (110), et dans lequel la détermination des réglages d'exposition automatique est automatiquement initiée une fois que la présence d'au moins un objet au sein d'une partie prédéterminée du champ de vision de la caméra est automatiquement détectée, l'objet comprenant la carte de référence de couleurs
c) la capture, en utilisant la caméra (120), d'au moins une image comprenant la scène (126) de l'étape b),
○ dans lequel les réglages d'exposition automatique déterminés de l'étape b) sont utilisés
ii) détermination de la concentration de l'analyte dans l'échantillon en utilisant l'au moins une image d'au moins une partie du champ de test de réactif (120) de la bandelette de test optique (118) ayant l'échantillon appliqué sur celle-ci, et au moins une partie de la pluralité de champs de référence de couleurs (112) différents.

2. Procédé selon l'une quelconque des revendications précédentes, dans lequel le dispositif mobile (128) comprend un écran (134), et le procédé comprend en outre dans l'étape i) l'étape de :
○ fourniture d'un guidage visuel sur l'écran (134) pour positionner la caméra (130) par rapport à un objet, l'objet comprenant au moins une partie de la bandelette de test optique (118) et/ou au moins une partie de la carte de référence de couleurs (110), pour déterminer les réglages d'exposition automatique et/ou pour capturer l'au moins une image.

3. Procédé selon l'une quelconque des revendications précédentes, dans lequel la carte de référence de couleurs et/ou la bandelette de test comprend au moins un marqueur de position (122).

4. Procédé selon la revendication précédente, dans lequel la détermination des réglages d'exposition automatique est initiée automatiquement dans le cas où il est déterminé que la caméra (130) est dans une position définie par rapport à la carte de référence de couleurs (110) en se basant sur l'au moins un marqueur de position (122).

5. Procédé selon l'une quelconque des deux revendications précédentes, dans lequel la position de la caméra (130) par rapport à la carte de référence de couleurs (110) en se basant sur l'au moins un marqueur de position (122) est déterminée et la zone de mesure d'exposition est réglée en fonction de la position déterminée.

6. Procédé selon l'une quelconque des revendications précédentes, dans lequel le champ de test de réactif (120) est dans une position définie par rapport à la carte de référence de couleurs (110) pendant une ou les deux parmi la détermination des réglages d'exposition automatique pour la caméra (130) et la capture de l'au moins une image.

7. Procédé selon l'une quelconque des revendications précédentes, dans lequel au moins 5 %, en particulier au moins 10 % ou au moins 15 % de la scène dans la zone de mesure d'exposition réglée est constituée d'au moins une partie du ou des champs d'arrière-plan de gris (114).

8. Procédé selon l'une quelconque des revendications précédentes, dans lequel le procédé de l'étape i) comprend en outre :
d) la détermination d'au moins un élément d'informations d'admissibilité, dans lequel l'élément d'informations d'admissibilité indique l'admissibilité dans le cas où chaque pixel ou au moins un groupe de pixels prédéterminé correspondant aux champs de référence de couleurs (112) dans l'au moins une image présente des valeurs de luminosité dans des limites acceptables, par exemple entre 50 et 240, ou entre 80 et 220.
